# EUROPEAN PATENT APPLICATION

(11) **EP 2 859 850 A1**
(43) Date of publication of application: **15.04.2015**
(21) Application number: 14179463.6
(22) Date of filing: 01.08.2014
(51) Int. Cl.: A61B 8/00, F16M 11/04

(54) **Control panel for ultrasonic diagnostic apparatus**

(30) Priority: 14.10.2013 KR 20130121801
(71) Applicant: Samsung Medison Co., Ltd., Gangwon-do 250-870 (KR)
(72) Inventor: Woo, Kyeong Gu, Gyeonggi-do (KR); Ko, Jae Yong, Seoul (KR)
(74) Representative: Schmid, Wolfgang

(57) **Abstract**

Disclosed herein is a control panel for an ultrasonic diagnostic apparatus. The control panel includes a first housing, a second housing disposed under the first housing, plural bearing balls provided between the first housing and the second housing and configured to support the first housing so that the first housing is spaced apart from the second housing, and a brake unit configured to prevent movement of the first housing by restricting rotation of the bearing balls or to allow movement of the first housing by releasing restriction of rotation of the bearing balls.

## Description

### BACKGROUND

### 1. Field

Embodiments of the present invention relate to a control panel for an ultrasonic diagnostic apparatus.

### 2. Description of the Related Art

In general, ultrasonic diagnostic apparatuses direct ultrasonic signals from a body surface of an object to a desired region inside a body, and obtain an image related to a monolayer of soft tissue or bloodstream using the ultrasonic signals reflected from the desired region.

An ultrasonic diagnostic apparatus comprises a main body, a probe to transmit an ultrasonic signal to an object to be examined and receive an echo signal reflected from the object, a display unit mounted to an upper portion of the main body and configured to display an image generated based upon the echo signal reflected from the object, and a control panel disposed in front of the display unit and configured to allow a user to manipulate the ultrasonic diagnostic apparatus.

### SUMMARY

It is an aspect of the present invention to provide a control panel for an ultrasonic diagnostic apparatus capable of planar movement and rotational movement.

Additional aspects of the invention will be set forth in part in the description which follows and, in part, will be obvious from the description, or may be learned by practice of the invention.

In accordance with one aspect of the present invention, a control panel for an ultrasonic diagnostic apparatus includes a first housing, a second housing disposed under the first housing, plural bearing balls provided between the first housing and the second housing and configured to support the first housing so that the first housing is spaced apart from the second housing, and a brake unit configured to prevent movement of the first housing by restricting rotation of the bearing balls or to allow movement of the first housing by releasing restriction of rotation of the bearing balls.

The first housing may include a separation-preventing part protruding from an edge portion of a bottom surface of the first housing, and the separation-preventing part may prevent the first housing from being separated from the second housing.

The second housing may include plural concave parts to receive the plural bearing balls therein.

The brake unit may restrict rotation of one of the plural bearing balls and may release restriction of rotation of the other bearing balls, thereby allowing the first housing to rotate about the bearing ball which is restricted in rotation.

The brake unit may be provided between the first housing and the second housing, or provided in the second housing.

The brake unit may include one or more magnetic holders, and the bearing balls may be restricted in rotation by magnetic force of the magnetic holders. Restriction of rotation of the bearing balls may be released by applying electric current to the magnetic holder.

The bearing balls may include a material which is magnetized when magnetic force is applied thereto, and the first housing may include a fixing layer disposed at a bottom surface thereof, which is made of a material which is magnetized when magnetic force is applied thereto.

The brake unit may include one or more brakes, and the bearing balls may be restricted in rotation by frictional force of the brakes.

Each of the brakes may include a power element to generate power, a contact element to generate frictional force by contacting the bearing balls, and a transmission element, one end of which is coupled to a rear surface of the contact element and the other end of which is coupled to the power element. The transmission element may make the contact element move forward and backward using power generated from the power element.

Restriction of rotation of the bearing balls may be released by separating the contact element from the bearing balls using power generated from the power element.

The bearing balls may be coated with a material capable of increasing frictional force, and the first housing may include a fixing layer to increase frictional force, which is disposed at a bottom surface of the first housing.

As described above, the control panel for an ultrasonic diagnostic apparatus according to the present invention achieves both planar movement and rotational movement with a simple structure.

In addition, the control of movement and restriction of the control panel may be easily achieved using magnetic force.

In addition, the control of movement and restriction of the control panel may be easily achieved using frictional force.

### BRIEF DESCRIPTION OF THE DRAWINGS

These and/or other aspects of the invention will become apparent and more readily appreciated from the following description of the embodiments, taken in conjunction with the accompanying drawings of which:
FIG. 1 is a perspective view illustrating an ultrasonic diagnostic apparatus according to an embodiment of the present invention;
FIG. 2 is a front view schematically illustrating the ultrasonic diagnostic apparatus to explain a control panel according to an embodiment of the present invention;
FIG. 3 is a plan view schematically illustrating the ultrasonic diagnostic apparatus to explain the control panel according to an embodiment of the present invention;
FIG. 4 is a view to explain bearing balls of the control panel according to an embodiment of the present invention;
FIGS. 5A and 5B are a sectional view schematically illustrating the control panel to explain an exemplary embodiment of a brake unit which operates based upon magnetic force; and
FIGS. 6A and 6B are a sectional view schematically illustrating the control panel to explain another exemplary embodiment of the brake unit which operates based upon frictional force.

### DETAILED DESCRIPTION

Now, preferred embodiments of the present invention will be described in detail with reference to the accompanying drawings so that the above and other aspects of the present invention will be easily understood and realized by those skilled in the art. In the following description, a detailed description of known functions and configurations incorporated herein will be omitted when it may make the subject matter of the present invention rather unclear.

It is also to be understood that the terms used in the following description are defined taking into consideration the functions obtained in accordance with the present invention. The definitions of these terms should be determined based on the whole content of this specification because they may be changed in accordance with the option of a user or a usual practice. Unless otherwise defined, the terms used herein have the same meanings as commonly understood by those skilled in the art.

Further, a variety of specific elements such as constituent elements are shown in the following description. The description of such elements has been made only for a better understanding of the present invention. Those skilled in the art will appreciate that various modifications, additions, and substitutions to the specific elements are possible, without departing from the scope and spirit of the invention as disclosed in the accompanying claims.

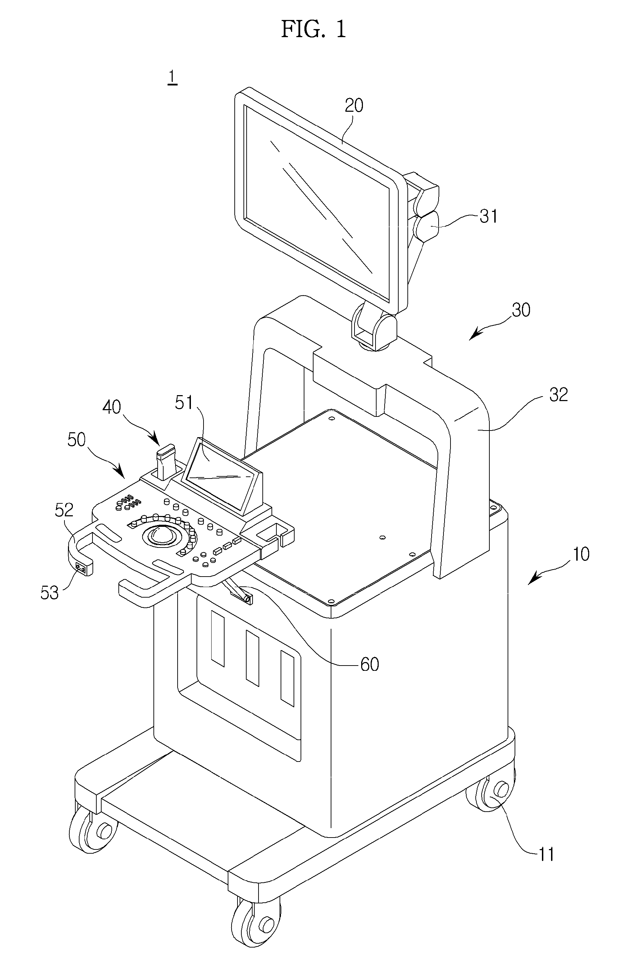

FIG. 1 is a perspective view illustrating an ultrasonic diagnostic apparatus according to an embodiment of the present invention.

As shown in FIG. 1, an ultrasonic diagnostic apparatus 1 comprises a main body 10, a display unit 20, a connection unit 30, a probe 40 and a control panel 50.

The main body 10 is configured to perform various operations to output an ultrasonic image according to a user command. Casters 11 may be mounted to a bottom surface of the main body 10, by which a user may move the main body 10 conveniently.

The display unit 20 is disposed above the main body 10 and displays data transmitted from the main body 10. For instance, the display unit 20 may display an ultrasonic diagnosis result transmitted from the main body 10 or display a screen indicating settings of the ultrasonic diagnostic apparatus 1 or a screen to set operating conditions.

The connection unit 30 is disposed between the main body 10 and the display unit 20 in order to connect the main body 10 and the display unit 20 to each other. The connection unit 30 may include a connection bracket 31 coupled to the display unit 20 and a support part 32 coupled to the main body 10. A space is created between the main body 10 and the display unit 20 due to the support part 32, in which medical supplies necessary for ultrasonic diagnosis may be stowed.

The probe 40 is configured to transmit an ultrasonic signal to an object to be examined and receive an echo signal reflected from the object. The echo signal reflected from the object may be transmitted to the main body 10.

A user inputs a control command of the ultrasonic diagnostic apparatus 1 through the control panel 50. The control panel 50 may be provided with an auxiliary display unit 51 at a portion thereof. The control panel 50 may be further provided with a handle 52 which protrudes forward from a front surface of the control panel 50 so that a user can grasp the same. The handle 52 is used to allow planar movement of the control panel 50.

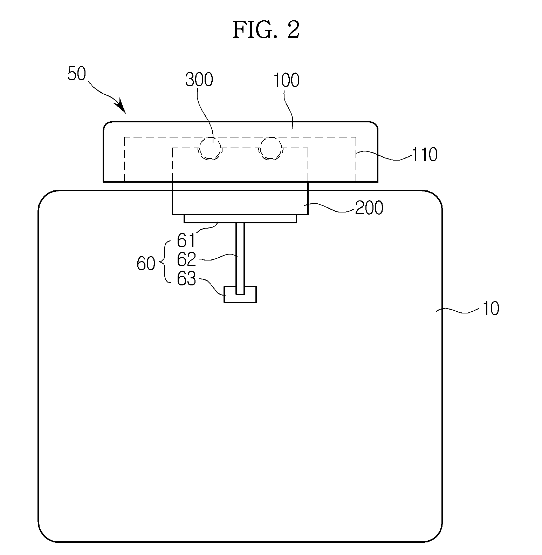

The control panel 50 may include an input device 53 through which a user inputs a command for planar movement of the control panel 50. Accordingly, the control panel 50 is in a stationary state when a command for planar movement is not input, but capable of planar movement when a command for planar movement is input. Hereinafter, operation of the control panel 50 will be described in detail with reference to FIGS. 2 through 6.

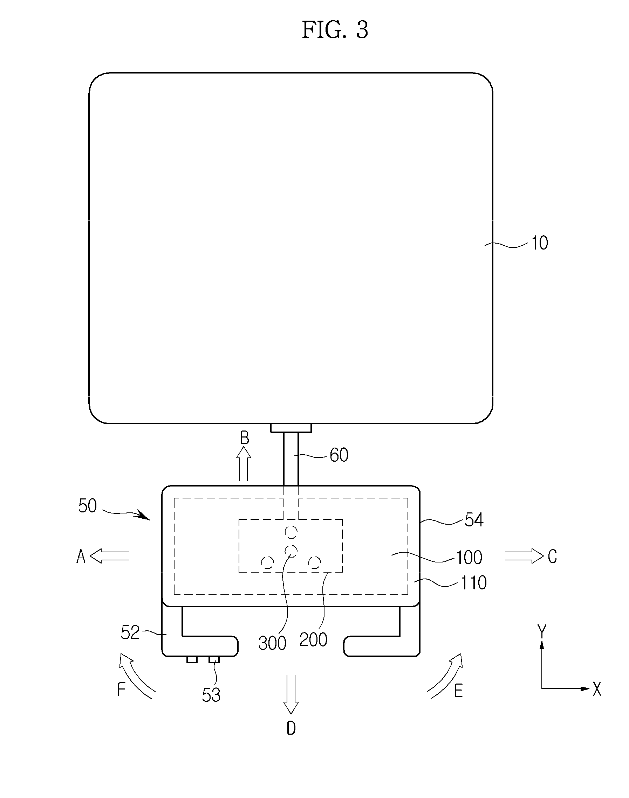

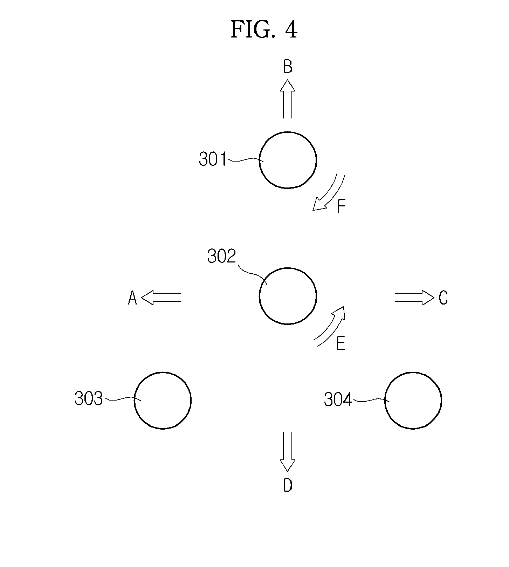

FIG. 2 is a front view schematically illustrating the ultrasonic diagnostic apparatus to explain the control panel according to an embodiment of the present invention, FIG. 3 is a plan view schematically illustrating the ultrasonic diagnostic apparatus to explain the control panel according to an embodiment of the present invention, and FIG. 4 is a view to explain bearing balls of the control panel according to an embodiment of the present invention.

Referring to FIGS. 2 and 3, the main body 10 and the control panel 50 are connected by a fixing unit 60. The fixing unit 60 includes a first fixing part 61, a connection part 62 and a second fixing part 63. The first fixing part 61 is coupled to a bottom surface of the control panel 50, and the second fixing part 63 is coupled to the main body 10. One end portion of the connection part 62 is coupled to a portion of the first fixing part 61, and the other end portion of the connection part 62 is coupled to a portion of the second fixing part 63. The control panel 50 is supported by the above-structured fixing unit 60.

The control panel 50 includes a first housing 100, a second housing 200 and bearing balls 300.

The handle 52, which is configured such that a user may grasp the same, may be provided at a front surface of the first housing 100. A user grasping the handle 52 may carry out planar movement of the first housing 100.

The input device 53 may be provided at a portion of the handle 52. A user may input a command for movement or restriction of the first housing 100 through the input device 53. For example, a user may input a command for planar movement or rotational movement of the first housing 100 through the input device 53.

The first housing 100 is positioned on the second housing 200. The first housing 100 may be provided with plural buttons at an upper surface thereof, through which a user inputs a control command of the ultrasonic diagnostic apparatus.

The first housing 100 may include a separation-preventing part 110. The separation-preventing part 110 serves to prevent separation of the first housing 100 and the second housing 200 from each other.

In detail, the separation-preventing part 110 may have a shape protruding downward from an edge portion of a bottom surface of the first housing 100. The separation-preventing part 110 protruding downward may restrict planar movement of the first housing 100. That is, as illustrated by dotted lines in FIG. 3, the separation-preventing part 110 is formed along the edge portion of the bottom surface of the first housing 100. If the first housing 100 undergoes planar movement until the separation-preventing part 110 comes into contact with the second housing 200, further planar movement of the first housing 100 is restricted. Accordingly, the separation-preventing part 110 prevents separation of the first housing 100 and the second housing 200 from each other.

For example, if the first housing 100 moves in a direction B (refer to FIG. 3) until the separation-preventing part 110 formed at a left edge portion of the first housing 100 comes into contact with the second housing 200, further planar movement of the first housing 100 is restricted.

The second housing 200 is positioned under the first housing 100. The first fixing part 61 of the fixing unit 60 is coupled to a bottom surface of the second housing 200. The second housing 200 is formed with concave parts at an upper surface thereof, in which the bearing balls 300 are received.

The bearing balls 300 are disposed between the first housing 100 and the second housing 200. In detail, a lower portion of each of the bearing balls 300 is received in each of the concave parts formed at the second housing 200. An upper portion of each of the bearing balls 300 is in contact with a bottom surface of the first housing 100. Accordingly, the bearing balls 300 support the first housing 100 so that the first housing 100 is spaced apart from the second housing 200.

Further, the bearing balls 300 serve to reduce frictional resistance when the first housing 100 undergoes planar movement, thereby enabling a user to easily move the first housing 100. That is, when the first housing 100 moves, the bearing balls 300 rotate in the concave parts and reduce resistance against planar movement of the first housing 100.

Additionally, by controlling rotation of the bearing balls 300, the movement of the first housing 100 may be controlled. In other words, if all the bearing balls 300 are not allowed to rotate, the first housing 100 may be kept in a fixed state due to high resistance. If all the bearing balls 300 are allowed to rotate, the first housing 100 may be changed to a movable state.

Referring to FIGS. 3 and 4, plural bearing balls 300 are provided between the first housing 100 and the second housing 200. If all the bearing balls 300 are not allowed to rotate, planar movement of the first housing 100 cannot be achieved due to high resistance.

However, if all the bearing balls 300 are allowed to rotate, resistance against movement is reduced and accordingly planar movement of the first housing 100 may be easily achieved by a user. In detail, the first housing 100 may undergo planar movement in various directions A, B, C and D by a user as shown in the drawings just until the separation-preventing part 110 comes into contact with the second housing 200. As soon as the separation-preventing part 110 contacts the second housing 200, movement of the first housing 100 is restricted.

In addition, rotational movement of the first housing 100 may be achieved by a user. For example, if rotation of a bearing ball 302 which is located in a center position of the plural bearing balls 300 is not allowed, the first housing 100 may rotate counterclockwise (direction E) about a rotating axis which is set by the bearing ball 302 which causes relatively high resistance. Additionally, if rotation of a bearing ball 301 which is located near the edge portion is not allowed, the first housing 100 may rotate counterclockwise (direction E) about a rotating axis which is set by the bearing ball 301 which causes relatively high resistance.

As described above, by controlling rotation of each of the bearing balls 300, planar movement or rotational movement of the control panel 50 is controlled.

Referring to FIGS. 1 through 4, rotation of the bearing balls 300 is controlled by a brake unit. In detail, the brake unit may keep each of the bearing balls 300 provided at the control panel from rotating. If rotation of the bearing balls 300 is restricted by the brake unit, movement of the first housing 100 is also restricted.

In addition, the brake unit may also release restriction of rotation of the bearing balls 300. In detail, if a user inputs a command for movement through the input device 53, the brake unit releases restriction of rotation of the bearing balls 300. The bearing balls 300 which are free from restriction can freely rotate in the concave parts of the second housing. Accordingly, if external force is applied to the first housing, the bearing balls 300 rotate and thus the first housing moves.

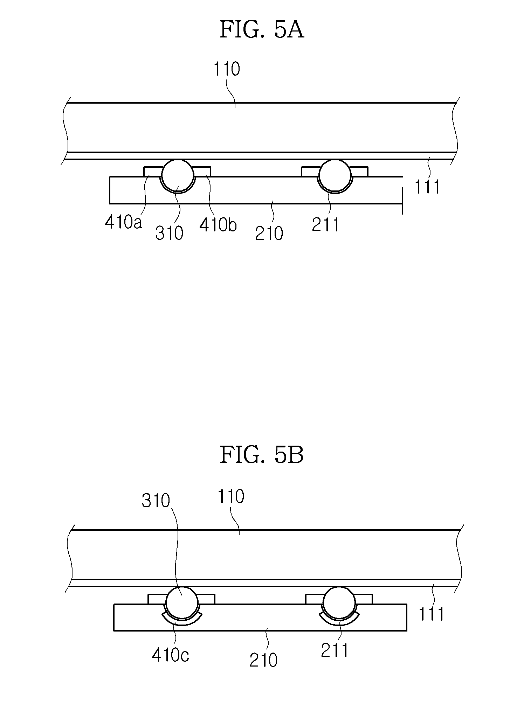

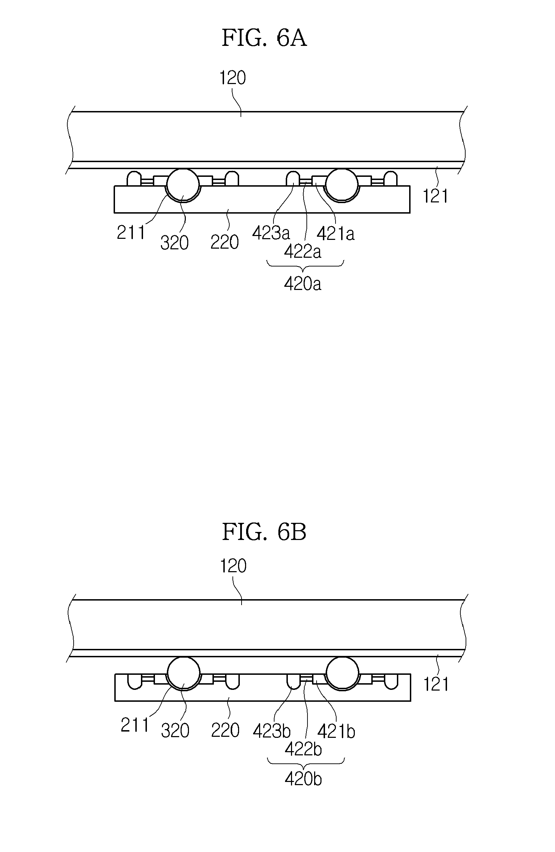

Hereinafter, exemplary embodiments of the brake unit will be explained with reference to FIGS. 5A, 5B, 6A and 6B.

FIGS. 5A and 5B are a sectional view schematically illustrating the control panel to explain an exemplary embodiment of the brake unit which operates based upon magnetic force. The brake unit may control movement or restriction of the first housing using magnetic force.

Referring to FIGS. 5A and 5B, the brake unit may operate to restrict rotation of the bearing balls 300 using magnetic force. If the bearing balls 300 are restricted in rotation by the brake unit, the first housing is kept stationary.

The brake unit may be a magnetic holder 410. The magnetic holder 410 has a feature of having magnetic force at a normal state but losing magnetic force when electric current flows therethrough. That is, the bearing balls 300 are fixed by magnetic force of the magnetic holder 410. If electric current flows through the magnetic holder 410, the magnetic holder 410 loses magnetic force and thus the bearing balls 300 can rotate. Accordingly, the movement or restriction of the first housing 110 is controlled by controlling electric current applied to the magnetic holder 410.

Alternatively, the brake unit may be an electromagnet holder. The electromagnet holder has a feature of creating magnetic force when electric current flows therethrough. Therefore, when electric current is applied to the electromagnet holder, the bearing balls 300 are not allowed to rotate. Because the control panel is normally in a stationary state, it is desirable to use the magnetic holder 410 as the brake unit. Accordingly, the brake unit is the magnetic holder 410 in the following description.

The bearing balls 310 are received in the concave parts 211. The bearing balls 310 may be rotated in the concave parts 211. Such bearing balls 310 support the first housing 110.

As shown in FIG. 5A, the magnetic holders 410a and 410b may be disposed between the first housing 110 and the second housing 210. Additionally, plural magnetic holders 410a and 410b may be provided for each of the bearing balls 310.

Alternatively, as shown in FIG. 5B, the magnetic holders 410c may be disposed in the second housing 210. In detail, the magnetic holders 410c may be provided at a portion of the concave parts 211.

The bearing balls 310 may include a material which is magnetized by magnetic force generated from the magnetic holders 410, so that the magnetic holders 410 restrict rotation of the bearing balls 310 using magnetic force. For example, the bearing balls 310 may include a material, such as steel, nickel, cobalt or the like, which is magnetized when magnetic force is applied thereto.

The first housing 110 may include a fixing layer 111. The fixing layer 111 may be disposed at a bottom surface of the first housing 110. The fixing layer 111 may be made of a material which is also magnetized by the magnetized bearing balls 310. For example, the fixing layer 111 may be a thin film layer including a material, such as steel, nickel, cobalt or the like, which is magnetized when magnetic force is applied thereto. Accordingly, attractive force is also exerted between the fixing layer 111 and the bearing balls 310, thereby securely fixing the first housing 110.

In detail, the bearing balls 310 are magnetized by magnetic force of the magnetic holders 410. Accordingly, attractive force is exerted between the magnetized bearing balls 310 and the magnetic holders 410, and rotation of the bearing balls 310 is restricted.

Further, the fixing layer 111 is magnetized by the magnetized bearing balls 310. Accordingly, attractive force is also exerted between the bearing balls 310 and the fixing layer 111, and the first housing 110 is fixed to the bearing balls 310.

If electric current is applied to the magnetic holders 410, restriction of rotation of the bearing balls 300 is released. In detail, if electric current is applied to the magnetic holders 410, the magnetic holders 410 lose magnetic force. If the magnetic holders 410 lose magnetic force, attractive force is no longer exerted between the bearing balls 310 and the magnetic holders 410. Additionally, attractive force is no longer exerted between the bearing balls 310 and the fixing layer 111.

Accordingly, if electric current flows through the magnetic holder 410, the bearing balls 310 are allowed to rotate. In such a state, if a user grasps the handle 52 (refer to FIG. 1) and applies proper force to move the control panel, the bearing balls 310 rotate and the first housing 110 moves.

In addition, the control panel may be controlled such that the first housing 110 undergoes only rotational movement by partially fixing the bearing balls 310. In detail, one of the bearing balls 310 is restricted in rotation by magnetic force of the magnetic holders 410 corresponding thereto, but restriction of rotation of the other bearing balls 310 is released by applying electric current to the magnetic holders 410 corresponding thereto. In other words, magnetic force exerted on the other bearing balls 310 is removed, and the other bearing balls 310 are allowed to rotate. In such a state, if a user applies force to the first housing 110, the first housing 110 may rotate about the bearing ball 310 which is restricted in rotation.

FIGS. 6A and 6B are a sectional view schematically illustrating the control panel to explain another exemplary embodiment of the brake unit which operates based upon frictional force. The brake unit may control movement or restriction of the first housing using frictional force.

Referring to FIGS. 6A and 6B, the brake unit may operate to restrict rotation of the bearing balls using frictional force.

The brake unit may be a brake 420, which generates frictional force by contact with the bearing balls 300 and restricts rotation of the bearing balls 300. If the bearing balls 300 are restricted in rotation, the first housing 120 supported by the bearing balls 300 is kept in a stationary state.

The brake 420 includes a contact element 421, a transmission element 422 and a power element 423.

The contact element 421 is configured to restrict rotation of the bearing balls 320 by contact with the same. That is, rotation of the bearing balls 320 is restricted by frictional force with the contact element 421. The contact element 421 may move forward and backward by the transmission element 422. In detail, the contact element 421 moves forward toward the bearing balls 320 to restrict rotation thereof, or moves backward from the bearing balls 320 to allow rotation thereof.

One end of the transmission element 422 is coupled to a rear surface of the contact element 421, and the other end of the transmission element 422 is coupled to the power element 423. The transmission element 422 makes the contact element 421 move forward and backward using power generated from the power element 423.

The power element 423 is configured to generate power. For example, the power element 423 may be a step motor which is capable of controlling rotation. The power element 423 and the transmission element 422 may be engaged with a structure of converting rotational movement into linear movement.

As shown in FIG. 6A, the brake 420a may be disposed between the first housing 120 and the second housing 220. Alternatively, as shown in FIG. 6B, the brake 420b may be disposed in the second housing 220. In detail, the brake 420b may be provided at a portion of the concave part 211. Plural brakes 420 may be provided for each of the bearing balls 320.

In order to restrict rotation of the bearing balls 320 using frictional force, the bearing balls 320 may be coated with a material capable of increasing frictional force. Here, the material capable of increasing frictional force means a material having a high coefficient of friction. Further, the bearing balls 320 may have a rough surface to increase frictional force. By increasing frictional force exerted on the bearing balls 320 as described above, the first housing 120 may be more securely kept in a stationary state.

The first housing 120 may include a fixing layer 121. The fixing layer 121 may be disposed at a bottom surface of the first housing 120. The fixing layer 121 is configured to increase frictional force between the bearing balls 320 and the first housing 120. In detail, the fixing layer 121 may be formed by coating the bottom surface of the first housing 120 with a material having a high coefficient of friction. Further, the fixing layer 121 may have a rough surface to increase frictional force. By increasing frictional force exerted on the bearing balls 320 as described above, the first housing 120 may be more securely kept in a stationary state.

In more detail, rotation of the bearing balls 320 is restricted by frictional force with the contact elements 421 of the plural brakes. Accordingly, movement of the first housing 120 is also restricted by frictional force with the bearing balls 320 which are restricted in rotation.

If the contact elements 421 move away from the bearing balls 320 by power generated from the power elements 423, the frictional force restricting rotation of the bearing balls 320 is removed and accordingly the bearing balls 320 are allowed to rotate. In such a state, if a user grasps the handle 52 (refer to FIG. 1) and applies proper force to move the control panel, the bearing balls 320 rotate and the first housing 120 moves.

In addition, the control panel may be controlled such that the first housing 120 undergoes only rotational movement by partially fixing the bearing balls 320. In detail, if the brake unit is controlled so that frictional force is exerted on one of the bearing balls 320 and the frictional force exerted on the other bearing balls 320 is removed, the first housing 120 is allowed to rotate about the bearing ball 320 on which frictional force is exerted. In other words, if a user grasps the handle 52 (refer to FIG. 1) and applies force to the first housing 120, the first housing 120 may rotate about the bearing ball 320 on which frictional force is exerted.

Although a few embodiments of the present invention have been shown and described, it would be appreciated by those skilled in the art that changes may be made in these embodiments without departing from the principles and spirit of the invention, the scope of which is defined in the claims and their equivalents.

## Claims

1. A control panel for an ultrasonic diagnostic apparatus comprising:
a first housing;
a second housing disposed under the first housing;
plural bearing balls provided between the first housing and the second housing and configured to support the first housing so that the first housing is spaced apart from the second housing; and
a brake unit configured to prevent movement of the first housing by restricting rotation of the bearing balls or to allow movement of the first housing by releasing restriction of rotation of the bearing balls.

2. The control panel for an ultrasonic diagnostic apparatus according to claim 1, wherein the second housing includes plural concave parts to receive the plural bearing balls therein.

3. The control panel for an ultrasonic diagnostic apparatus according to claim 1, wherein the brake unit includes one or more magnetic holders, and
the bearing balls are restricted in rotation by magnetic force of the magnetic holders.

4. The control panel for an ultrasonic diagnostic apparatus according to claim 3, wherein restriction of rotation of the bearing balls is released by applying electric current to the magnetic holder.

5. The control panel for an ultrasonic diagnostic apparatus according to claim 3, wherein the bearing balls include a material which is magnetized when magnetic force is applied thereto.

6. The control panel for an ultrasonic diagnostic apparatus according to claim 3, wherein the first housing includes a fixing layer disposed at a bottom surface thereof, and
the fixing layer is made of a material which is magnetized when magnetic force is applied thereto.

7. The control panel for an ultrasonic diagnostic apparatus according to claim 1, wherein the brake unit includes one or more brakes, and
the bearing balls are restricted in rotation by frictional force of the brakes.

8. The control panel for an ultrasonic diagnostic apparatus according to claim 7, wherein each of the brakes includes:
a power element to generate power;
a contact element to generate frictional force by contacting the bearing balls;
and
a transmission element, one end of which is coupled to a rear surface of the contact element and the other end of which is coupled to the power element,
wherein the transmission element makes the contact element move forward and backward using power generated from the power element.

9. The control panel for an ultrasonic diagnostic apparatus according to claim 8, wherein restriction of rotation of the bearing balls is released by separating the contact element from the bearing balls using power generated from the power element.

10. The control panel for an ultrasonic diagnostic apparatus according to claim 7, wherein the bearing balls are coated with a material capable of increasing frictional force.

11. The control panel for an ultrasonic diagnostic apparatus according to claim 7, wherein the first housing includes a fixing layer to increase frictional force, which is disposed at a bottom surface of the first housing.

12. The control panel for an ultrasonic diagnostic apparatus according to claim 1, wherein the first housing includes a separation-preventing part protruding from an edge portion of a bottom surface of the first housing, and
the separation-preventing part prevents the first housing from being separated from the second housing.

13. The control panel for an ultrasonic diagnostic apparatus according to claim 1, wherein the brake unit restricts rotation of one of the plural bearing balls and releases restriction of rotation of the other bearing balls, thereby allowing the first housing to rotate about the bearing ball which is restricted in rotation.

14. The control panel for an ultrasonic diagnostic apparatus according to claim 1, wherein the brake unit is provided between the first housing and the second housing.

15. The control panel for an ultrasonic diagnostic apparatus according to claim 1, wherein the brake unit is provided in the second housing.
